# EUROPÄISCHE PATENTSCHRIFT

(11) **EP 0 523 003 B1**
(45) Veröffentlichungstag und Bekanntmachung des Hinweises auf die Patenterteilung: **30.08.1995**
(21) Anmeldenummer: 92810488.4
(22) Anmeldetag: 25.06.1992
(51) Int. Cl.: C07D 495/06, C08K 5/45, H01B 1/12

(54) **Radikalkationensalze von Tetrathiotetracen und Kupferchlorid, deren Herstellung und deren Verwendung**
Cation-radical salts of tetrathiotetracene and copper chloride, their preparation and use
Sels cation-radicalaires de tétrathiotétracène et de chlorure de cuivre, leur préparation et leur utilisation

(30) Priorität: 02.07.1991 CH 1952/91
(43) Veröffentlichungstag der Anmeldung: 13.01.1993
(73) Patentinhaber: CIBA-GEIGY AG, 4002 Basel (CH)
(72) Erfinder: Hilti, Bruno, Dr., CH-4054 Basel (CH); Minder, Ernst, CH-4450 Sissach (CH); Mayer, Carl W., Dr., CH-4125 Riehen (CH); Klingert, Bernd, Dr., W-7854 Inzlingen (DE)

(56) Entgegenhaltungen:
- EP-A- 0 285 564
- DE-A- 2 641 742
- DE-A- 3 510 092
- I.F. SHCHEGOLEV et al.: "Cation-Radical Salts of TTT and TSeT", in: "Extended Linear Chain Compounds", Ed.: J.S. Miller, vol. 2, 1982, New York; pages 393-394
- CHEMICAL ABSTRACTS, vol. 94, no. 4, 26. Januar 1981, Columbus, Ohio, US; abstract no. 22829v, M. MASSON ET AL. 'Photooxidation of tetrathiatetracene ...' Seite 489 ;

## Beschreibung

Die Erfindung betrifft Radikalkationensalze aus Tetrathiotetracen (nachfolgend mit TTT abgekürzt) und CuCl₂, ein Verfahren zu deren Herstellung durch Oxidation von TTT in organischen Lösungsmitteln mit wasserfreiem CuCl₂, CuCl₂-Aquokomplexen oder CuCl₂-Lösungsmittelkomplexen, und deren Verwendung als elektrischer Leiter zum Beispiel zur antistatischen Ausrüstung von Polymeren oder zur Herstellung von elektrisch leitenden Filmen, Folien, Beschichtungen oder Formkörpern.

In einem Übersichtsartikel beschreiben I. F. Shchegolev et al. in Extended Linear Chain Compounds, Vol. 2, Editor J. S. Miller, Plenum Press New York, Seiten 393 und 394 (1982) Radikalkationenkomplexe aus TTT und CuBr₂ sowie aus Tetraselenotetracen und CuCl₂. Die elektrische Leitfähigkeit dieser Komplexe ist selbst bei Einkristallen relativ niedrig. Die spezifische Leitfähigkeit a bei 300 K wird mit 10⁻² bis 10⁻³ Ω⁻¹cm⁻¹ angegeben. Bei Radikalkationkomplexen dieser Art werden im allgemeinen dann hohe elektrische Leitfähigkeiten beobachtet, wenn das Anion Brom oder Iod enthält. Solche Komplexe weisen aber eine geringe thermische Beständigkeit auf. Die I₃ Komplexe ihre Herstellung und Verwendung sind beispielsweise in der DE 26 41 742 beschrieben.

In Chem. Phys. Lett. 1980, 76(1), 92-5 wird die Photooxidation von Tetrathiotetracenen in Halogenkohlenwasserstofflösungen beschrieben, die zu Halogen-Radikalkationensalzen führt.

In der EP-A-285 564 sind leitfähige Zusammensetzungen beschrieben, die ein Polymer, ein Tetrathio- oder Tetraselenotetracen und gegebenenfalls einen Elektronenakzeptor enthalten.

Aus der DE-35 10 092 sind in 2 bzw. 2,3-Stellung substituierte Tetrathio- oder Tetraselenotetracene, ihre Herstellung und Verwendung für elektrisch leitende Schichten bekannt.

Es wurde nun gefunden, dass Radikalkationsalze aus TTT und CuCl₂ überraschend eine bis zu etwa zwei Grössenordnungen höhere elektrische Leitfähigkeit besitzen, zudem thermisch wesentlich stabiler sind und damit auch bei höheren Temperaturen verarbeitet werden können. Diese Salze kristallisieren in Form von Nadeln und können zum Aufbau von Netzwerken aus Kristallnadeln in einer Polymermatrix verwendet werden. Diese Salze unterscheiden sich in Ihrer Zusammensetzung auch überraschend und deutlich von den zuvor erwähnten bekannten Komplexen.

Ein Gegenstand der Erfindung sind Radikalkationensalze mit der Zusammensetzung der Formel I 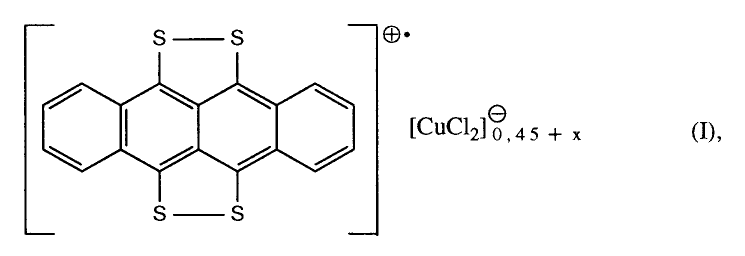
worin x einen Zahlenwert von -0,1 bis +0,2 bedeutet.

Der Zahlenwert von x hängt im wesentlichen von den Herstellungsbedingungen ab, hauptsächlich vom verwendeten Lösungsmittel, der Art der Ausgangsprodukte und deren Mengenverhältnisse. Der Zahlenwert von x beträgt bevorzugt -0,05 bis +0,15, besonders bevorzugt -0,05 bis +0,1, und insbesondere bevorzugt -0,05 bis +0,05. Ganz besonders bevorzugt sind Radikalkationensalze der Formel I, worin x den Zahlenwert -0,02 aufweist.

Ein anderer Gegenstand der Erfindung ist ein Verfahren zur Herstellung von Radikalkationensalzen der Formel I, das dadurch gekennzeichnet ist, dass man wasserfreies CuCl₂, einen CuCl₂-Aquokomplex oder einen CuCl₂-Lösungsmittelkomplex in einem organischen Lösungsmittel in einer Menge von 0,3 bis 0,8 Mol pro Mol Tetrathiotetracen mit Tetrathiotetracen umsetzt.

Das Kupferdichlorid beziehungsweise die Kupferdichloridhydrate oder Kupferdichlorid-Lösungsmittelkomplexe werden bevorzugt in einer Menge von 0,35 bis 0,6 Mol pro Mol Tetrathiotetracen eingesetzt.

Bei Verwendung von wasserfreiem Kupferdichlorid kann dem Reaktionsgemisch Wasser zugegeben werden, zum Beispiel in Form wasserhaltiger Lösungsmittel.

Lösungsmittelkomplexe von Kupferdichlorid und polaren aprotischen oder polaren protischen Lösungsmitteln sind in grosser Anzahl bekannt Es können monomere, dimere und polymere Komplexe verwendet werden. Geeignete Lösungsmittel sind hauptsächlich solche mit Heteroatomen wie zum Beispiel Sauerstoff, Schwefel, Phosphor und Stickstoff. Einige Beispiele sind Ether (Diethylether, Dibutylether, Ethylenglykoldimethyl- oder -diethylether), Ester und Lactone (Essigsäureethylester, γ-Butyrolacton), Sulfone (Dimethylsulfon, Tetramethylensulfon) und Amine (Pyridin, α-Pyridon, α-Methylpyridin, Ethylendiamin, N,N-Dimethylethylendiamin, 1-(β-Aminoethyl)pyridin, 1-(β-Methylaminoethyl)-pyridin.

Die Reaktion wird in Gegenwart eines inerten Lösungsmittels durchgeführt. Geeignete Lösungsmittel, die alleine oder als Mischung von Lösungsmitteln eingesetzt werden können, sind zum Beispiel aliphatische und aromatische Kohlenwasserstoffe, wie z.B. Pentan, Hexan, Cyclohexan, Methylcyclohexan, Benzol, Nitrobenzol, Toluol, Xylol und Biphenyl; Alkohole wie z.B. Methanol, Ethanol, Propanol und Butanol; Ether wie z.B. Diethylether, Diethylenglykoldimethylether, Ethylenglykoldimethylether, Diphenylether, Tetrahydrofuran und Dioxan; Halo genkohlenwasserstoffe wie z.B. Methylenchlorid, Chloroform, 1,1,2,2-Tetrachlorethan und Chlorbenzol; Ester und Lactone wie z.B. Essigsäureethylester, Butyrolacton oder Valerolacton; Carbonsäureamide und Lactame wie z.B. Dimethylformamid, Dimethylacetamid und N-Methylpyrrolidon. Sofern die Reaktanden schwerlöslich sind, können sie in Form von Suspensionen in einem Lösungsmittel eingesetzt werden.

Das erfindungsgemässe Verfahren wird vorteilhaft bei erhöhten Temperaturen durchgeführt, zum Beispiel bei 30 bis 300 °C, bevorzugt bei 50 bis 250 °C. Man kann zum Beispiel so vorgehen, dass man zu einer heissen Lösung von Tetrathiotetracen eine Lösung der Kupferverbindung zugibt und dann das Reaktionsgemisch abkühlen lässt. Der gebildete kristalline Niederschlag wird danach abfiltriert und gegebenenfalls durch Auswaschen gereinigt und getrocknet. Wenn grössere Kristallite gewünscht sind, kann man eine diffusionskonnollierte Reaktion durchführen, indem man die festen Reaktanden getrennt in den Vorratsbehältern eines Reaktionsgefässes vorlegt und dann mit einem Lösungsmittel überschichtet. Die Radikalkationsalze werden in hoher Reinheit erhalten.

Die erfindungsgemässen Radikalkationensalze sind thermisch sehr stabil und können auch bei hohen Temperaturen in Kunststoffe eingearbeitet werden. Man erzielt hierbei auf Grund der hohen elektrischen Leitfähigkeit (σ des gepressten Pulvers von etwa 0,4 bis 2,0 Ω⁻¹·cm⁻¹) in Formkörpern eine gute antistatische Ausrüstung. Die Fähigkeit dieser Radikalkationensalze zur Bildung nadelförmiger Kristalle erschliesst die Möglichkeit, Formteile wie zum Beispiel Beschichtungen oder freitragende Filme mit hoher elektrischer Leitfähigkeit herzustellen, die bis zu etwa 40 % der Leitfähigkeit der kristallinen erfindungsgemässen Radikalkationensalze betragen kann. Die Herstellung kann in einfacher Weise durch Zugabe des Tetrathiotetracens und der Kupferverbindung zu einem schmelzflüssigen Kunststoff erfolgen, der nach der Formgebung abgekühlt wird, oder durch Zugabe zu einer Kunststofflösung und Verdampfen des Lösungsmittels nach der Verarbeitung. Hierbei werden dichte und feinmaschige Netzwerke von Kristallnadeln in einer Polymermatrix ausgebildet, die zu einer hohen elektrischen Leitfähigkeit führen.

Ein weiterer Gegenstand der Erfindung ist eine Zusammensetzung, enthaltend a) ein duroplastisches, thermoplastisches oder strukturell vernetztes Polymer und b) ein Radikalkationensalz der Formel I in Form eines Netzwerks aus Kristallnadeln in der Polymermatrix.

Das Radikalkationensalz kann in einer Menge von 0,01 bis 30 Gew.-%, bevorzugt 0,01 bis 20 Gew.-%, besonders bevorzugt 0,01 bis 10 Gew.-% und insbesondere bevorzugt 0,1 bis 5 Gew.-% enthalten sein, bezogen auf die Zusammensetzung.

Bei den thermoplastischen Polymeren kann es sich zum Beispiel um die folgenden Polymeren, Copolymeren bzw. Mischungen von diesen Polymeren handeln:
1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. EthylenPropylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen/Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere oder Ethylen-Acrylsäure-Copolymere und deren Salze (Ionomere), sowie Terpolymere von Ethylen mit Propylen und einen, Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Kohlenwasserstoffharze (z.B. C₅-C₉) inklusive hydrierte Modifikationen davon (z.B. Klebrigmacherharze).
4. Polystyrol, Poly-(p-methylstyrol), Poly-(α-methylstyrol).
5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Strol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsaureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von α,β-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polvalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit thermoplasrischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestem und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylylendiamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme'').
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly- 1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherketone.
20. Polyether aus Diglycidylverbindungen, zum Beispiel Diglycidylethern und Diolen, z. B. aus Bisphenol-A-Diglycidylether und Bisphenol-A.
21. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
22. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate` POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Bevorzugte Thermoplaste sind Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol.

Bei den Duroplasten und strukturell vernetzten Polymeren kann es sich zum Beispiel um folgende Polymere handeln:
1. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
2. Trocknende und nicht-trocknende Alkydharze.
3. Ungesättigte Polyesterharze, die sich von Copolyestern gesättigter und ungesättigter Dicarbonsäuren mit mehrwertigen Alkoholen, sowie Vinylverbindungen als Vernetzungs-mittel ableiten, wie auch deren halogenhaltige, schwerbrennbare Modifikationen.
4. Vernetzbare Acrylharze, die sich von substituierten Acrylsäureestern ableiten, wie z.B. von Epoxyacrylaten, Urethan-acrylaten oder Polyester-acrylaten.
5. Alkydharze, Polyesterharze und Acrylatharze, die mit Melaminharzen, Harnstoffharzen, Polyisocyanaten oder Epoxidharzen vernetzt sind.
6. Kautschuk auf der Basis von vernetzten Polydienen, zum Beispiel Butadien oder Isopren; Silikonkautschuk.
7. Epoxidharze, die sich von Polyepoxiden ableiten, z.B. von Bisglycidylethern von Polyolen oder von cycloaliphatischen Diepoxiden, und die gegebenenfalls einen Härter als Vernetzungsmittel enthalten, oder die unter Verwendung von Härtungsbeschleunigern thermisch oder durch Einwirkung von Strahlung vernetzt sind.

Unter den vernetzten Polymeren sind vernetzte Epoxidharze bevorzugt, denen als Polyepoxide bevorzugt Glycidylverbindungen mit durchschnittlich zwei Epoxidgruppen im Molekül zu Grunde liegen. Als Glycidylverbindungen kommen vor allen, solche mit zwei an ein Heteroatom (z.B. Schwefel, vorzugsweise Sauerstoff oder Stickstofß gebundenen Glycidylgruppen, β-Methylglycidylgruppen oder 2,3-Epoxycyclopentylgruppen in Frage; genannt seien insbesondere Bis-(2,3-epoxycyclopentyl)-ether; Diglycidylether von mehrwertigen aliphatischen Alkoholen, wie 1,4-Butandiol, oder Polyalkylenglykolen, wie Polypropylenglyhole; Diglycidylether von cycloaliphatischen Polyolen, wie 2,2-Bis-(4-hydroxycyclohexyl)-propan; Diglycidylether von mehrwertigen Phenolen, wie Resorcin, Bis-(p-hydroxyphenyl)-methan, 2,2-Bis-(p-hydroxyphenyl)-propan (=Diomethan), 2,2-Bis-(4'-hydroxy-3',5'-dibromphenyl)-propan, 1,3-Di-(p-hydroxyphenyl)-ethan; Di-(β-methylglycidyl)-ether der oben angeführten zweiwertigen Alkohole oder zweiwertigen Phenole; Diglycidylester von Dicarbonsäuren, wie Phthalsäure, Terephthalsäure, Δ₄-Tetrahydrophthalsäure und Hexahydrophthalsäure; N,N-Diglycidylderivate von primären Aminen und Amiden und heterocyclischen, zwei N-Atome enthaltenden Stickstoffbasen, und N,N'-Diglycidylderivate von disekundären Diamiden und Diaminen, wie N,N-Diglycidylanilin, N,N-Diglycidyltoluidin, N,N-Diglycidyl-p-aminophenyl-methyl-ether, N,N'-Dimethyl-N,N'-diglycidyl-bis-(p-aminophenyl)-methan; N',N''-Diglycidyl-N-phenyl-isocyanurat; N,N'-Diglycidylethylenharnstoff; N,N'-Diglycidyl-5,5-dimethyl-hydantoin, N,N'-Diglycidyl-5-isopropyl-hydantoin, N,N-Methylen-bis-(N',N'-diglycidyl-5,5-dimethylhydan-toin), 1,3-Bis-(N-glycidyl-5,5-dimethylhydantoin)-2-hydroxypropan; N,N'-Diglycidyl-5,5-dimethyl-6-isopropyl-5,6-dihydro-uracil, Triglycidylisocyanurat.

Eine bevorzugte Gruppe von Epoxidharzen sind glycidylierte Novolake, Hydantoine, Aminophenole, Bisphenole und aromatische Diamine oder cycloaliphatische Epoxidverbindungen. Besonders bevorzugte Epoxidharze sind glycidylierte Kresolnovolake, Bisphenol-A- und Bisphenol-F-diglycidylether, Hydantoin-N,N'-bisglycid, p-Aminophenoltriglycid, Diaminodiphenylmethantenaglycid, Vinylcyclohexendioxid, 3,4-Epoxycyclohexylmethyl-3,4-epoxycyclohexancarboxylat oder Mischungen hiervon.

Geeignet sind auch vorreagierte Addukte solcher Epoxidverbindungen mit Epoxidhärtern, zum Beispiel ein Addukt aus Bisphenol-A-diglycidylether und Bisphenol-A, oder mit Oligoestern mit zwei terminalen Carboxylguppen und Epoxiden vorreagierte Addukte.

Als Härter für Epoxidharze kommen saure oder basische Verbindungen in Frage. Als geeignete Härter seien zum Beispiel genannt: mehrwertige Phenole (Resorcin, 2,2-Bis-(4-hydroxyphenyl)-propan) oder Phenol-Formaldehyd-Harze; mehrbasische Carbonsäuren und ihre Anhydride, z. B. Phthalsäureanhydrid, Tetrahydrophthalsäureanhydrid, Hexahydrophthalsäureanhydrid, 4-Methylhexahydrophthalsäureanhydrid, 3,6-Endomethylen-tetrahydrophthalsäreanhydrid, 4-Methyl-3,6-endomethylen-tetrahydrophthalsäurehydrid (Methylnadicanhydrid), 3,4,5,6,7,7-Hexachlor-endomethylen-tetrahydrophthalsäureanhydrid, Bernsteinsäureanhydrid, Adipinsäureanhydrid, Trimethyladipinsäureanhydrid, Sebacinsäureanhydrid, Maleinsäureanhydrid, Dodecylbemsteinsäureanhydrid, Pyromellitsäuredianhydrid, Trimellitsäureanhydrid, Benzophenontetracarbonsäuredianhydrid, oder Gemische solcher Anhydride.

Eine bevorzugte Gruppe von Härtern sind Novolake und Polycarbonsäureanhydride.

Die Epoxidharze können auch zusätzlich mit Härtungsbeschleunigern oder nur mit thermischen Härtungskatalysatoren gehärtet werden. Beispiele sind 3-Ethyl-4-methylimidazol, Triamylammoniumphenolat); Mono- oder Polyphenole (Phenol, Diomenthan, Salicylsäure); Bortrifluorid und seine Komplexe mit organischen Verbindungen wie z. B. Bortrifluorid-Etherkomplexe und BortrifluorldAmin-Komplexe (BF₃-Monoethylamin-Komplex); Phosphorsäure und Triphenylphosphit.

Härtungsbeschleuniger und Katalysatoren werden üblicherweise in einer Menge von 0,1 bis 10 Gew.-% zugegeben, bezogen auf das Epoxidharz. Härter für Epoxidharze werden im allgemeinen in äquimolaren Mengen verwendet, bezogen auf die Epoxidgruppen und funktionellen Gruppen eines Härters.

Der erfindungsgemässen Zusammensetzung können weitere Additive zur Verbesserung der Verarbeitungseigenschaften, der mechanischen, elektrischen und thermischen Eigenschaften, der Oberflächeneigenschaften und der Lichtstabilität einverleibt sein, zum Beispiel feinteilige Füllstoffe, Verstärkerfüllstoffe, Weichmacher, Gleit- und Entformungsmittel, Haftvermittler, Antistatika, Antioxidantien, Wärme- und Lichtstabilisatoren, Pigmente und Farbstoffe.

Eine bevorzugte Ausführungsform der erfindungsgemässen Zusammensetzung ist dadurch gekennzeichnet, dass sie als Formkörper, Filme, Folien, Fasern, oder als Beschichtungen auf mindestens einer Oberfläche eines Substrats ausgebildet ist.

Die Herstellung der erfindungsgemässen Zusammensetzung kann nach in der Kunststofftechnik bekannten Verfahren erfolgen. Bei Formgebungsverfahren für Polymere, zum Beispiel Giessen, Pressverfahren, Spritzgiessen und Extrusion, kann das Radikalkationensalz selbst unter Bildung von Suspensionen, oder TTT und die Kupferverbindung gemeinsam der Polymerschmelze, mindestens einem Ausgangsstoff für Duroplaste, oder getrennt je einem Ausgangsstoff (zum Beispiel dem Epoxidharz und dem Härter) unter Bildung von Lösungen oder Suspensionen zugegeben werden, wobei nach der Formgebung das Radikalkationensalz während des Abkühlens in Form von Nadeln auskristallisiert beziehungsweise ausfällt, die ein Netzwerk in einer Polymermatrix bilden.

In einer besonders bevorzugten Ausführungsform ist die erfindungsgemässe Zusammensetzung als Film oder Folie oder als Beschichtung auf mindestens einer Oberfläche eines Substrats ausgebildet. Zur Herstellung solcher Ausführungsformen wird zum Beispiel ein thermoplastisches Polymer oder mindestens ein Ausgangsprodukt für einen Duroplasten oder ein strukturell vernetztes Polymer in einem gegebenenfalls inerten Lösungsmittel gelöst oder suspendiert, danach mittels bekannter Beschichtungstechniken auf ein gegebenenfalls vorerwärmtes Substrat aufgetragen und danach unter Erwärmen das Lösungsmittel entfernt, wobei vernetzbare Mischungen dann noch ausgehärtet werden können. Freitragende Filme und Folien werden durch Ablösen vom Substrat oder mittels Extrusionsverfahren erhalten.

Geeignete Substrate sind zum Beispiel Glas, Metalle, Kunststoffe, mineralische und keramische Werkstoffe, Holz und Papier. Die Substrate können beliebige äussere Formen aufweisen; es kann sich zum Beispiel um Formkörper, Fäden, Fasern, Gewebe, Stangen, Rohre, Bänder, Folien, Platten, Walzen oder Gehäuse handeln.

Geeignete Beschichtungsverfahren sind zum Beispiel Streichen, Walzen, Rakeln, Giessen, Schleudergiessen, Vorhanggiessen und Sprayen. Besonders bevorzugte Verfahren sind Sprayverfahren, weil zum einen sehr dünne und gleichmässige Schichten mit im wesentlichen isotropen, sehr feinmaschigen und homogenen Netzwerken aus Kristallnadeln der Radikalkationensalze erhältlich sind, und zum anderen die Grösse der Kristallnadeln und die Maschenweite der Netzwerke durch die Tröpfchengrösse kontrolliert werden kann, selbst wenn man Suspensionen sprüht.

Geeignete inerte Lösungsmittel für Polymere beziehungsweise Ausgangsprodukte für Polymere sind zum Beispiel polare und bevorzugt aprotische Lösungsmittel, die alleine oder in Mischungen aus mindestens zwei Lösungsmitteln verwendet werden können. Beispiele sind: Ether (Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Sulfoxide (Dimethylsulfoxid), Sulfone (Dimethylsulfon, Diethylsulfon, Trimeihylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol) und Nitrile (Acetonitril, Propionitril).

Die Beschichtungsverfahren können zum Beispiel so durchgeführt werden, dass man die einzelnen Bestandteile getrennt löst und erst vor dem gewählten Beschichtungsverfahren vereinigt. Man kann aber auch die Bestandteile in zwei Lösungen, zum Beispiel Polymer und TTT oder Kupferverbindung sowie Kupferverbindung beziehungsweise TTT herstellen, oder alle Bestandteile in einer Lösung vereinigen. Im letzten Fall können die Radikalkationensalze schon vor der Beschichtung auskristallisieren, was aber praktisch keinen Einfluss auf die gewünschte Qualität der Beschichtung hat.

Die Lösungen werden zweckmässig erwärmt, zum Beispiel auf 50 bis 250 °C. Es ist vorteilhaft, auch das Substrat zu erwärmen, um die Entfernung des Lösungsmittels zu beschleunigen, die im allgemeinen bei Temperaturen von 50 bis 200 °C vorgenommen wird, bis die Beschichtung trocken ist. Sofern die Beschichtungen zu frei tragenden Filmen oder Folien abgelöst werden sollen, kann das Substrat vor der Beschichtung mit Antihaftmitteln behandelt werden.

In einer Herstellvariante für Beschichtungen kann man auch die erfindungsgemässen Radikalkationensalze, die als Kristallnadeln ausgebildet sind, in einer Lösung eines Polymeren oder Ausgangsstoffen für Duroplaste suspendieren, dann ein Substrat beschichten und danach das Lösungsmittel entfernen und gegebenenfalls zur Bildung der Duroplaste aushärten. Ferner ist es möglich, pulvrige Trockenmischungen aus Polymerpulvern oder festen Ausgangsstoffen für Duroplaste und den Radikalkationensalzen herzustellen und diese in gegebenenfalls elektrostatischen Beschichtungsverfahren zu Schichten auf Substraten zu verarbeiten. Auch bei diesen Varianten werden Netzwerke von Kristallnadeln in einer Polymermatrix erhalten.

Es ist auch möglich, reine Schichten von Netzwerken aus Kristallnadeln der CT-Komplexe auf einem Substrat herzustellen, indem man zum Beispiel Lösungen oder Suspensionen der CT-Komplexe in einem Lösungsmittel auf einem Substrat aufbringt und danach das Lösungsmittel verdampft. Solche Schichten können zur Erhöhung der elektrischen Leitfähigkeit elektrochemisch metallisiert werden, zum Beispiel mit Cu, Pt oder Pd. Es kann zweckmässig sein, solche reine Schichten mit einer Schutzschicht aus einem Polymer zu beschichten.

Die Schichtdicken können in einem breiten Rahmen variieren, je nach Wahl des Beschichtungsverfahrens. Mit Sprayverfahren können sehr dünne Schichten erhalten werden, während man mit Streich- und Giessverfahren auch dicke Schichten erzielen kann. Die Schichtdicken können zum Beispiel 0,01 bis 5000 »m, bevorzugt 0,1 bis 1000 »m und besonders bevorzugt 0,1 bis 500 »m betragen.

Die erfindungsgemässe Zusammensetzung ist je nach Wahl des Polymers opak oder transparent und sie weist hervorragende elektrische Eigenschaften auf. So weisen die Beschichtungen und Formkörper überraschend eine ausgezeichnete Entladungsfähigkeit auf, die für heterogene Materialien sonst schwierig oder gar nicht zu erreichen ist. Die Zusammensetzung eignet sich daher besonders zur Verwendung als antistatisch ausgerüstete Formteile für die elektrostatische Abschirmung von Bauteilen oder zur Verwendung als antistatisch ausgerüstete Formkörper. Die hohen elektrischen Leitfähigkeiten ermöglichen ferner die Verwendung als elektrische Leiter, zum Beispiel als Elektroden für Displayelemente oder elektronische Bauteile. Die Zusammensetzungen weisen auch hervorragende mechanische Festigkeiten und mechanische Gebrauchseigenschaften auf.

Die nachfolgenden Beispiele erläutern die Erfindung näher.

### A) Herstellungsbeispiele

Beispiel A1:60 mg (TTT) werden in 33 g γ-Butyrolacton unter Argonatmosphäre bei 180 °C Badtemperatur mit Rühren gelöst (grüne Lösung). Anschliessend wird eine gelbe Lösung von 15,3 mg CuCl₂·2 H₂O in 5 g γ-Butyrolacton zugegeben. Die Lösung wird sofort dunkelviolett. Der Rührer und die Heizung des Oelbads werden abgestellt und die Reaktionslösung wird darin im Ölbad abgekühlt. Dabei kristallisieren Nadeln aus (Ausbeute 57 mg). Der spezifische Widerstand der Kristalle beträgt 0.5 bis 1 Ω·cm. Die Röntgenstrukturanalyse zeigt, dass es sich um das Radikalkationensalz der Zusammensetzung TTT(CuCl₂)_{0,45} handelt.

Beispiele A2 bis A8: Es wird wie in Beispiel A1 verfahren, aber ohne Schutzgasatmosphäre und anstelle von CuCl₂·2 H₂O werden wasserfreies CuCl₂ oder Lösungsmittelkomplexe verwendet.

Beispiel A2: Lösungsmittelkomplex: CuCl₂(Dimethylsulfoxid)₂, Zusammensetzung TTT(CuCl₂)_{0,44}, σ=0,7273 Ω⁻¹·cm⁻¹.

Beispiel A3: Lösungsmittelkomplex: [CuCl₂(α-Pyrrolidon)]₂, Zusammensetzung TTT(CuCl₂)_{0,43}, σ=1,629 Ω⁻¹·cm⁻¹.

Beispiel A4: Lösungsmittelkomplex: [CuCl₂(Tetramethylensulfon)] (Polymer), Zusammensetzung TTT(CuCl₂)_{0,44}, σ=1,3238 Ω⁻¹·cm⁻¹.

Beispiel A5: Lösungsmittelkomplex: CuCl₂(α-Picolin)₂, Zusammensetzung TTT(CuCl₂)_{0,42}, σ=1,02 Ω⁻¹·cm⁻¹.

Beispiel A6: Lösungsmittelkomplex: [CuCl₂(Butyrolacton)], (Polymer)Zusammensetzung TTT(CuCl₂)_{0,59}, σ=1,597 Ω⁻¹·cm⁻¹.

Beispiel A7: Lösungsmittelkomplex: [CuCl₂(Butyrolacton)] (Polymer), die Lösung enthält 0,5 % Wasser, Zusammensetzung TTT(CuCl₂)_{0,51}, σ=0,6954 Ω⁻¹·cm⁻¹.

Beispiel A8: Verwendung von wasserfreiem CuCl₂ anstelle von einem Lösungsmittelhomplex; Zusammensetzung TTT(CuCl₂)_{0,44}, σ=1,1 Ω⁻¹·cm⁻¹.

### B) Anwendungsbeispiele

Beispiel B1: 9,0 mg TTT und 0,6 g Polycarbonat werden bei 160 °C in 18 g γ-Butyrolacton gelöst und mit einer Lösung von 2,1 mg CuCl₂·2 H₂O in 2 g γ-Butyrolacton vermischt. Das Reaktionsgemisch wird auf eine vorgeheizte Glasplatte gesprüht (Sprühbedingungen: Glasdüse mit einem Durchmesser von 1 mm, Treibgas Argon, Abstand von Sprühdüse zur Glasplatte ca. 15 cm). Nach Abdampfen des Lösungsmittels bei 130 °C bleibt eine 8 »m dicke Schicht mit einem feinen Nadelnetzwerk aus elektrisch leitenden Kristallnadeln von TTT(CuCl₂)_{0,48} in einer Polycarbonatmatrix zurück. Der spezifische Widerstand beträgt 2,4·10³ Ωcm.

Beispiel B2: 9,0 mg Tetrathiotetracen und 0,6g Polycarbonat werden bei 150 °C in 16g Anisol gelöst. Dazu werden 2,0 mg CuCl₂·2 H₂O gelöst in 2 g γ-Butyrolacton gegeben. Das Radikalkationensalz kristallisiert sofort in kleinen Nadeln aus. Das Reaktionsgemisch wird auf eine vorgeheizte Glasplatte gesprüht (Sprühbedingungen wie bei Beispiel B1) und das Lösungsmittel bei 100 °C verdampft. Der gebildete Polycarbonatfilm ist transparent und enthält ein feines, dichtes, elektrisch leitendes Nadelnetzwerk. Der spezifische Widerstand beträgt 6·10³ Ωcm.
Messung der Oberflächenentladung:
Die Oberflächenspannung von etwa 200 V wird mit Hilfe eines goldbeschichteten Wolframdrahts von 50 Micrometer Durchmesser gebildet, der mit einer Spannung von ungefähr 3.4 kV aufgeladen wird. Diese Spannung ist so geregelt, dass der Strom bei konstant 20 nA pro cm Drahtlänge gehalten wird. Die Probe wird mit Silberpaste auf einen Glasträger geklebt und mit einer Kontaktspitze am Rand des Trägers elektrisch verbunden. Während eines Messverlaufs verschiebt sich der Träger 8 mm unter dem Coronadraht mit einer Geschwindigkeit von etwa 50 cm/s, und hält an dem Punkt an, wo die Kontaktspitze in einen geerdeten leitfähigen Schaum eintaucht. Die Probe liegt dann unter einem Feldmeter (Isoprobe Electrostatic Voltmeter 244, Monroe Electronics Inc.). Der Abfall der gemessenen Oberflächenspannung wird mit einem digitalen Oszilloskop gespeichert.Die Folien der Beispiele B1 und B2 werden mit diesem Test geprüft. Die Oberflächenspannung wird 0,5 Sekunden nach der Coronaaufladung gemessen. Ergebnis:

Beispiel B1: 2 ± 1 Volt; Beispiel B2: 2 ± 1 Volt.

Beispiel B3: 20,1 mg TTT und 2,5 g Polycarbonat werden bei 180 °C in 14,5 g γ-Butyrolacton gelöst. Dazu werden 5,5 mg CuCl₂·2H₂O gelöst in 1,5 g γ-Butyrolaeton, das 0,5 Gew.-% Wasser enthält, gegeben. Nach 30 Sekunden wird daraus auf einer Glasplatte mit einem Rakel ein Film mit einer Nassfilmdicke von 270 »m gezogen. Danach wird das Lösungsmittel bei 100 °C verdampft und der Film anschliessend bei 150 °C getrocknet. Der spezifische Widerstand des Films beträgt 500 bis 800 Ω·cm und die Filmdicke 23 »m.

Beispiel B4: 15,27 mg Tetrathiotetracen und 3,75 g eines Polyethers aus Bisphenol-A-Diglycidylether und Bisphenol-A werden bei 150 °C in 60 g Anisol gelöst. Nach etwa 30 Minuten werden 2,5 g einer Lösung von 3,75 mg CuCl₂·2 H₂O und 2% Wasser in 2,5 g γ-Butyrolaeton und 750 »l einer Anisollösung enthaltend 10% eines Polyurethanoligomers (Netzmittel) in Xylol zugegeben und vermischt. Das Reaktionsgemisch wird auf eine vorgeheizte Glasplatte gesprüht (Sprühbedingungen: Zweistoffdüse aus Stahl, Treibgas Argon, Abstand von Sprühdüse zur Glasplatte ca. 20 cm, Spraygeschwindigkeit 4 cm/s). Nach Abdanlpfen des Lösungsmittels bei 50 °C bleibt eine 5 »m dicke Schicht mit einem dichten Nidelnetzwerk aus elektrisch leitenden Kristallnadeln von TTT(CuCl₂)_{0,43} in einer Polyethermatrix zurück. Der spezifische Widerstand beträgt 2·10³ Ωcm.

Beispiel B5: Die Beschichtungen gemäss den Beispielen B1 und B3 werden 600 h bei 85% relativer Luftfeuchtigkeit und und 85 °C gehalten und der Widerstand nach der Vierpunktmethode gemessen. Der Widerstand bleibt praktisch unverändert.

## Patentansprüche

1. Radikalkationensalze mit der Zusammensetzung der Formel I 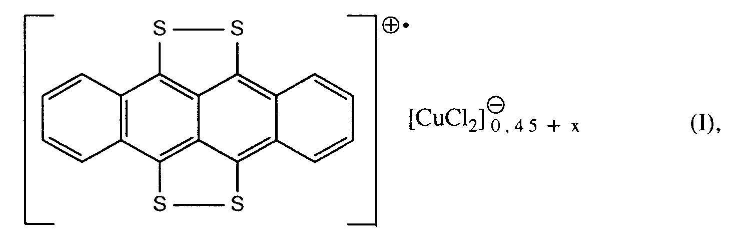 worin x einen Zahlenwert von -0,1 bis +0,2 bedeutet.

2. Radikalkationensalze gemäss Anspruch 1, worin in Formel I x einen Zahlenwert von -0,05 bis +0,15 bedeutet.

3. Radikalkationensalze gemäss Anspruch 1, worin in Formel I x einen Zahlenwert von -0,05 bis +0,1 bedeutet.

4. Radikalkationensalze gemäss Anspruch 1, worin in Formel I x einen Zahlenwert von -0,05 bis +0,05 bedeutet.

5. Radikalkationensalze gemäss Anspruch 1, worin in Formel I x einen Zahlenwert von -0,02 bedeutet.

6. Verfahren zur Herstellung von Radikalkationensalzen der Formel I gemäss Anspruch 1, dadurch gekennzeichnet, dass man wasserfreies CuCl₂, einen CuCl₂-Aquokomplex oder einen CuCl₂-Lösungsmittelkomplex in einem organischen Lösungsmittel in einer Menge von 0,3 bis 0,8 Mol pro Mol Tetrathiotetracen mit Tetrathiotetracen umsetzt.

7. Verfahren gemäss Anspruch 6, dadurch gekennzeichnet, dass das wasserfreie Kupferdichlorid, die Kupferdichloridaquokomplexe oder Kupferdichlorid-Lösungsmittelkomplexe in einer Menge von 0,35 bis 0,6 Mol pro Mol Tetrathiotetracen eingesetzt werden.

8. Verfahren gemäss Anspruch 6, dadurch gekennzeichnet, dass die Lösungsmittelkomplexe vom Kupferdichlorid Lösungsmittel mit Heteroatomen aus der Gruppe Sauerstoff, Schwefel, Phosphor und Stickstoff enthalten.

9. Verfahren gemäss Anspruch 6, dadurch gekennzeichnet, dass es bei erhöhten Temperaturen durchgeführt wird.

10. Verfahren gemäss Anspruch 9, dadurch gekennzeichnet, dass die Temperatur 30 bis 300 °C beträgt.

11. Zusammensetzung, enthaltend a) ein duroplastisches, thermoplastisches oder strukturell vernetztes Polymer und b) ein Radikalkationensalz der Formel I gemäss Anspruch 1 in Form eines Netzwerks aus Kristallnadeln in der Polymermatrix.

12. Zusammensetzung gemäss Anspruch 11, dadurch gekennzeichnet, dass das Radikalkationensalz in einer Menge von 0,01 bis 30 Gew.-% enthalten ist, bezogen auf die Zusammensetzung.

13. Zusammensetzung gemäss Anspruch 11, dadurch gekennzeichnet, dass es sich bei dem thermoplastischen Polymer um Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol.

14. Zusammensetzung gemäss Anspruch 11, dadurch gekennzeichnet, dass es sich bei dem duroplastischen Polymer um ein Epoxidharz handelt.

15. Zusammensetzung gemäss Anspruch 11, dadurch gekennzeichnet, dass sie als Formkörper, Filme, Folien, Fasern, oder als Beschichtungen auf mindestens einer Oberfläche eines Substrats ausgebildet ist.

16. Zusammensetzung gemäss Anspruch 15, dadurch gekennzeichnet, dass die Schichtdikke der Beschichtungen 0,01 bis 5000 »m beträgt.

17. Zusammensetzung gemäss Anspruch 15, dadurch gekennzeichnet, dass die Schichtdikke der Beschichtungen 0,1 bis 1000 »m beträgt.

18. Zusammensetzung gemäss Anspruch 11, worin in Formel I x einen Zahlenwert von -0,05 bis +0,15 bedeutet.

19. Zusammensetzung gemäss Anspruch 11, worin in Formel I x einen Zahlenwert von -0,05 bis +0,1 bedeutet.

20. Zusammensetzung gemäss Anspruch 11, worin in Formel I x einen Zahlenwert von -0,05 bis +0,05 bedeutet.

21. Zusammensetzung gemäss Anspruch 11, worin in Formel I x einen Zahlenwert von -0,02 bedeutet.

22. Verwendung der Zusammensetzung gemäss Anspruch 11 als antistatisch ausgerüstete Formteile für die elektrostatische Abschirmung von Bauteilen oder als antistatisch ausgerüstete Formkörper.

23. Verwendung der Zusammensetzung gemäss Anspruch 11 als elektrische Leiter.

## Claims

1. A radical cation salt having the composition of formula I 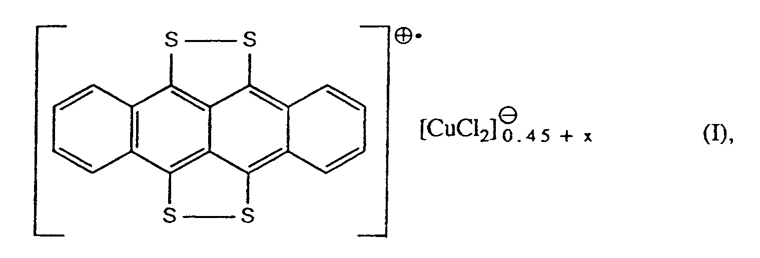 wherein x has a value from -0.1 to +0.2.

2. A radical cation salt according to claim 1, wherein the value of x in formula I is -0.05 to +0.15.

3. A radical cation salt according to claim 1, wherein the value of x in formula I is -0.05 to +0.1.

4. A radical cation salt according to claim 1, wherein the value of x in formula I is -0.05 to +0.05.

5. A radical cation salt according to claim 1, wherein the value of x in formula I is -0.02.

6. A process for the preparation of a radical cation salt of formula I according to claim 1, which comprises reacting anhydrous CuCl₂, a CuCl₂ aquo complex or a CuCl₂ solvent complex, in an organic solvent, in an amount of 0.3 to 0.8 mol per mol of tetrathiotetracene with tetrathiotetracene.

7. A process according to claim 6, wherein the anhydrous copper dichloride, the copper dichloride aquo complexes or copper dichloride solvent complexes are used in an amount of 0.35 to 0.6 mol per mol of tetrathiotetracene.

8. A process according to claim 6, wherein the solvent complexes of copper dichloride contain solvents containing hetero atoms selected from the group consisting of oxygen, sulfur, phosphorus and nitrogen.

9. A process according to claim 6 which is carried out at elevated temperatures.

10. A process according to claim 9, wherein the temperature is from 30 to 300 °C.

11. A composition comprising a) a thermosetting, thermoplastic or structurally crosslinked polymer, and b) a radical cation salt of formula I according to claim 1 in the form of a network of crystal needles in the polymer matrix.

12. A composition according to claim 11, which contains the radical cation salt in an amount of 0.01 to 30 % by weight, based on the composition.

13. A composition according to claim 11, wherein the thermoplastic polymer is selected from the group consisting of polyolefins, polystyrene, polyvinyl chloride, polyvinylidene chloride, polyvinylidene fluoride, polyacrylates, polymethacrylates, polyamides, polyesters, polycarbonates, aromatic polysulfones, aromatic polyethers, aromatic polyether sulfones, polyimides and polyvinyl carbazole.

14. A composition according to claim 11, wherein the thermosetting polymer is an epoxy resin.

15. A composition according to claim 11, which is shaped to mouldings, films, sheets, fibres or a coating on at least one surface of a substrate.

16. A composition according to claim 15, wherein the layer thickness of the coating is 0.01 to 5000 »m.

17. A composition according to claim 15, wherein the layer thickness of the coating is 0.1 to 1000 »m.

18. A composition according to claim 11, wherein the value of x in formula I is -0.05 to +0.15.

19. A composition according to claim 11, wherein the value of x in formula I is -0.05 to +0.1.

20. A composition according to claim 11, wherein the value of x in formula I is -0.05 to +0.05.

21. A composition according to claim 11, wherein the value of x in formula I is -0.02.

22. Use of a composition as claimed in claim 11 as antistatically treated moulded part for the electrostatic screening of components or as antistatically treated moulding.

23. Use of a composition as claimed in claim 11 as electric conductor.

## Revendications

1. Sels cation-radicalaires ayant la composition correspondant à la formule I 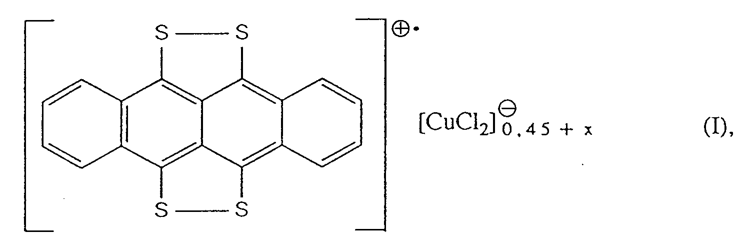 dans lesquels x est un nombre compris entre -0,1 et +0,2.

2. Sels cation-radicalaires selon la revendication 1, dans lesquels, dans la formule I, x est un nombre compris entre -0,05 et +0,15.

3. Sels cation-radicalaires selon la revendication 1, dans lesquels, dans la formule I, x est un nombre compris entre -0,05 et +0,1.

4. Sels cation-radicalaires selon la revendication 1, dans lesquels, dans la formule I, x est un nombre compris entre -0,05 et +0,05.

5. Sels cation-radicalaires selon la revendication 1, dans lesquels, dans la formule I, x est un nombre valant -0,02.

6. Procédé pour préparer des sels cation-radicalaires de formule I selon la revendication 1, caractérisé en ce qu'on fait réagir du CuCl₂ anhydre, un complexe de CuCl₂ et d'eau ou un complexe de CuCl₂ et d'un solvant dans un solvant organique en une quantité de 0,3 à 0,8 mole par mole de tétrathiotétracène, avec du tétrathiotétracène.

7. Procédé selon la revendication 6, caractérisé en ce qu'on utilise le dichlorure de cuivre anhydre, les complexes de dichlorure de cuivre et d'eau ou les complexes de dichlorure de cuivre et d'un solvant en une quantité de 0,35 à 0,6 mole par mole de tétrathiotétracène.

8. Procédé selon la revendication 6, caractérisé en ce que les complexes du dichlorure de cuivre avec un solvant contiennent des solvants comportant des hétéroatomes choisis parmi l'ensemble comprenant l'oxygène, le soufre, le phosphore et l'azote.

9. Procédé selon la revendication 6, caractérisé en ce qu'il est mis en oeuvre à haute température.

10. Procédé selon la revendication 9, caractérisé en ce que la température est de 30 à 300°C.

11. Composition contenant a) un polymère thermodurcissable, un polymère thermoplastique ou un polymère à réticulation structurale, et b) un sel cation-radicalaire de formule I selon la revendication 1 sous forme d'un réseau d'aiguilles cristallines dans la matrice polymère.

12. Composition selon la revendication 11, caractérisée en ce que le sel cation-radicalaire est présent en une quantité de 0,01 à 30 % en poids par rapport à la composition.

13. Composition selon la revendication 11, caractérisée en ce que, pour ce qui est du polymère thermoplastique, il s'agit de polyoléfines, de polystyrène, de poly(chlorure de vinyle), de poly(chlorure de vinylidène), de poly(fluorure de vinylidène), de polyacrylates, de polyméthacrylates, de polyamides, de polyesters, de polycarbonates, de polysulfones aromatiques, de polyéthers aromatiques, de polyéthersulfones aromatiques, de polyimides et de polyvinylcarbazole.

14. Composition selon la revendication 11, caractérisée en ce que, pour ce qui est du polymère thermodurcissable, il s'agit d'une résine époxyde.

15. Composition selon la revendication 11, caractérisée en ce qu'elle se présente sous forme d'objets moulés, de feuils, de feuilles, de fibres ou d'enductions sur au moins une surface d'un substrat.

16. Composition selon la revendication 15, caractérisée en ce que l'épaisseur de couche des enductions est de 0,01 à 5000 »m.

17. Composition selon la revendication 15, caractérisée en ce que l'épaisseur de couche des enductions est de 0,1 à 1000 »m.

18. Composition selon la revendication 11, dans laquelle, dans la formule I, x est un nombre compris entre -0,05 et +0,15.

19. Composition selon la revendication 11, dans laquelle, dans la formule I, x est un nombre compris entre -0,05 et +0,1.

20. Composition selon la revendication 11, dans laquelle, dans la formule I, x est un nombre compris entre -0,05 et +0,05.

21. Composition selon la revendication 11, dans laquelle, dans la formule I, x est un nombre valant -0,02.

22. Utilisation de la composition selon la revendication 11 comme pièces moulées antistatiques pour le blindage électrostatique de composants, ou comme objets moulés antistatiques.

23. Utilisation de la composition selon la revendication 11 comme conducteur électrique.
